# EUROPÄISCHE PATENTSCHRIFT

(11) **EP 0 500 083 B1**
(45) Veröffentlichungstag und Bekanntmachung des Hinweises auf die Patenterteilung: **13.09.1995**
(21) Anmeldenummer: 92102779.3
(22) Anmeldetag: 19.02.1992
(51) Int. Cl.: C07C 253/14, C07C 255/50

(54) **Verfahren zur Herstellung von 2,4-Dichlor-5-fluorbenzonitril**
Process for the preparation of 2,4-dichloro-5-fluorobenzonitrile
Procédé de préparation de 2,4-dichloro-5-fluorobenzonitrile

(30) Priorität: 20.02.1991 DE 4105187
(43) Veröffentlichungstag der Anmeldung: 26.08.1992
(73) Patentinhaber: HOECHST AKTIENGESELLSCHAFT, 65926 Frankfurt am Main (DE)
(72) Erfinder: Schach, Thomas, Dr., W-6238 Hofheim am Taunus (DE); Papenfuhs, Theodor, Dr., W-6000 Frankfurt am Main 50 (DE)

(56) Entgegenhaltungen:
- EP-A- 0 004 660
- EP-A- 0 433 124
- DE-B- 1 268 132
- PATENT ABSTRACTS OF JAPAN vol. 8, no. 71 (C-217)(1508) 1984 & JP-A-58 225 053 ( DAIKIN KOGYO K.K. )

## Beschreibung

Die vorliegende Erfindung betrifft ein verbessertes Verfahren zur Herstellung von 2,4-Dichlor-5-fluorbenzonitril durch Austausch des Bromatoms im 5-Brom-2,4-dichlorfluorbenzol gegen die Cyanidgruppe. Die genannte Verbindung und die daraus durch Hydrolyse der Nitrilgruppe erhältliche 2,4-Dichlor-5-fluorbenzoesäure stellen wichtige Zwischenprodukte zur Herstellung antibakteriell wirksamer Fluorchinolone dar.

Die Herstellung von 2,4-Dichlor-5-fluorbenzonitril ist bekannt aus CN 1,031,074. Bei der dort beschriebenen Synthese wird 2,4-Dichlor-5-fluornitrobenzol durch Eisenreduktion in das entsprechende Amin überführt (85 %ige Ausbeute). Durch Diazotierung und eine sich daran anschließende Cyan-Sandmeyer-Reaktion ist aus dem Amin das 2,4-Dichlor-5-fluorbenzonitril zugänglich, welche durch Hydrolyse in die entsprechende Carbonsäure überführt wird (70 %ige Ausbeute).

Nachteilig bei diesem bekannten Verfahren ist zum einen die schlechte Zugänglichkeit der verwendeten Ausgangsverbindung 2,4-Dichlor-5-fluornitrobenzol, zum anderen die mäßigen Ausbeuten an isoliertem Endprodukt, die, bezogen auf das eingesetzte 2,4-Dichlor-5-fluornitrobenzol, unter 60 % liegen.

Es bestand daher ein Bedürfnis nach einem verbesserten Verfahren, durch das die Herstellung von 2,4-Dichlor-5-fluorbenzonitril und daraus durch Hydrolyse der Nitrilgruppe die 2,4-Dichlor-5-fluorbenzoesäure, ausgehend von einer leicht zugänglichen Ausgangsverbindung, in hohen Ausbeuten ermöglicht wird.

Es wurde nun überraschenderweise gefunden, daß man 2,4-Dichlor-5-fluorbenzonitril in vorteilhafter Weise und in guten Ausbeuten herstellen kann, indem man 5-Brom-2,4-dichlorfluorbenzol mit etwa 10 bis etwa 300 Mol.-% Kupfer(I)-cyanid in Gegenwart von etwa 10 bis etwa 2000 Mol.-% eines polar aprotischen Lösungsmittels ausgenommen N-methyl pyrrolidon bei Temperaturen von etwa 100 bis etwa 250°C zum 2,4-Dichlor-5-fluorbenzonitril umsetzt. Das so erhaltene Nitril kann nach Entfernung des polaraprotischen Lösungsmittels mit der wäßrigen Lösung eines Metallhydroxids zu 2,4-Dichlor-5-fluorbenzoesäure hydrolysiert werden.

Das hierbei als Ausgangsverbindung eingesetzte 5-Brom-2,4-dichlorfluorbenzol ist durch Bromierung von 2,4-Dichlorfluorbenzol einfach und in 85 - 90 %iger Ausbeute zugänglich.

Hinsichtlich Einzelheiten der Verfahrensdurchführung sei noch folgendes ausgeführt:
Es ist zweckmäßig, das Brom-2,4-dichlorfluorbenzol zusammen mit dem Kupfer(I)-cyanid und dem polar aprotischen Lösungsmittel im Reaktionsgefäß vorzulegen, aufzuschmelzen und die Suspension auf Reaktionstemperatur zu bringen.

Um die gegen Ende der Brom-Austausch-Reaktion stark auftretenden Nebenreaktionen möglichst gering zu halten, wird das Kupfer(I)-cyanid besonders bevorzugt in Mengen von etwa 80 bis etwa 100 Mol.-%, bezogen auf 5-Brom-2,4-dichlorfluorbenzol, eingesetzt und die Reaktion bevorzugt bei Umsätzen von 75 bis 90 % abgebrochen.

Das erfindungsgemäße Verfahren kann in Gegenwart von Luftsauerstoff durchgeführt werden. Die Anwendung eines Schutzgases, wie beispielsweise Argon oder Stickstoff, ist möglich, wobei sich aber nur geringe Unterschiede zum Arbeiten ohne Schutzgasatmosphäre ergeben.

Falls das anfallende 2,4-Dichlor-5-fluorbenzonitril isoliert werden soll, so erfolgt die bevorzugte Aufarbeitung durch fraktionierte Destillation, wobei nichtumgesetzte Ausgangsverbindung zurückgewonnen werden kann.

Falls gewünscht, so kann das erfindungsgemäß erhaltene 2,4-Dichlor-5-fluorbenzonitril durch basische Hydrolyse mit Metallhydroxyden in die 2,4-Dichlor-5-fluorbenzoesäure überführt werden. Die Dauer der Hydrolyse beträgt hierbei zwischen etwa 0,5 und etwa 3 Stunden bei Temperaturen von etwa 80 bis etwa 100°C. Hierbei wird das vom Lösungsmittel befreite Rohprodukt (2,4-Diohlor-5-fluorbenzonitril) mit Alkali- oder Erdalkalimetallhydroxyden erhitzt und nach Abtrennen der Kupfersalze aus der wäßrigen Phase durch Sauerstellen mit einer anorganischen Säure ausgefällt.

Das erfindungsgemäße Verfahren wird durch das nachstehende Beispiel näher erläutert, ohne darauf beschränkt zu werden.

### Beispiel

In einen 500 ml Dreihalskolben mit Rückflußkühler und Blattrührer werden (1 mol) 243,89 g 5-Brom-2,4-dichlorfluorbenzol (0,9 mol) 80,61 g Kupfer(I)cyanid und (0,81 mol) 70 g Dimethylacetamid im Reaktionsgefäß vorgelegt und auf 150°C erhitzt. Unter guter Rührung wird die Reaktionssuspension für weitere 4 - 5 Stunden bei dieser Temperatur belassen. Anschließend wird auf 40 bis 50°C abgekühlt und die ausgefallenen Salze abgenutscht. Der Filterkuchen wird 3mal mit je 50 ml Methylenchlorid nachgewaschen und die vereinigten organischen Phasen zusammen mit der Mutterlauge fraktioniert destilliert. Man erhält neben 39,0 g 5-Brom-2,4-dichlorfluorbenzol 127,6 g (80,1 %) 2,4-Dichlor-5-fluorbenzonitril, bezogen auf umgesetztes 5-Brom-2,4-dichlorfluorbenzol, mit einer Reinheit (GC) >98 %.

Will man das so erhaltene 2,4-Dichlor-5-fluorbenzonitril in die 2,4-Dichlor-5-fluorbenzoesäure überführen, so wird die, wie vorstehend beschrieben, erhaltene Rohlösung im Vakuum vom Lösungsmittel und nicht umgesetztem 5-Brom-2,4-dichlorfluorbenzol befreit und mit 112 g (2,8 mol) Natriumhydroxyd in 1000 g Wasser für ca. 2 Stunden bei 100°C erhitzt, bis die Gasentwicklung beendet ist. Anschließend wird die Rohlösung mit konzentrierter Salzsäure auf pH 4,5 eingestellt und der ausgefallene Niederschlag abfiltriert. Durch weitere Zugabe von HCl bis auf und der ausgefallene Niederschlag abfiltriert. Durch weitere Zugabe von HCl bis auf pH 2,0 wird die gebildete 2,4-Dichlor-5-fluorlbenzoesäure ausgefällt, anschließend abgenutscht und dreimal mit je 50 ml Wasser gewaschen. Nach dem Trocknen erhält man 126,4 g (72 %) 2,4-Dichlor-5-fluorbenzoesäure mit einer Reinheit (GC) >99 %.

## Patentansprüche

1. Verfahren zur Herstellung von 2,4-Dichlor-5-fluorbenzonitril, dadurch gekennzeichnet, daß man 5-Brom-2,4-dichlorfluorbenzol mit etwa 10 bis etwa 300 Mol.-% Kupfer(I)-cyanid in Gegenwart von etwa 10 bis etwa 2000 Mol.-% eines polar aprotischen Lösungsmittels, ausgenommen N-Methylpyrrolidon, bei Temperaturen von etwa 100 bis etwa 250°C zum 2,4-Dichlor-5-fluorbenzonitril umsetzt.

2. Verfahren nach Anspruch 1, dadurch gekennzeichnet, daß man die Umsetzung mit Kupfer(I)-cyanid bei Temperaturen von etwa 130 bis etwa 190°C vornimmt.

3. Verfahren nach mindestens einem der Ansprüche 1 und 2, dadurch gekennzeichnet, daß man die Umsetzung mit Kupfer(I)-cyanid in Gegenwart von Tetramethylensulfon, Dimethylsulfoxid, Tetramethylensulfoxid, Dimethylacetamid oder Dimethylformamid als polar aprotischem Lösungsmittel vornimmt.

4. Verfahren nach mindestens einem der Ansprüche 1 bis 3, dadurch gekennzeichnet, daß man mit etwa 50 bis etwa 110 Mol.-% Kupfer(I)-cyanid umsetzt.

5. Verfahren nach mindestens einem der Ansprüche 1 bis 4, dadurch gekennzeichnet, daß man die Umsetzung mit Kupfer(I)-cyanid in Gegenwart von etwa 50 bis etwa 200 Mol.-% eines polar aprotischen Lösungsmittels umsetzt.

6. Verfahren nach mindestens einem der Ansprüche 1 bis 5, dadurch gekennzeichnet, daß man in Gegenwart eines Schutzgases arbeitet.

7. Verfahren nach mindestens einem der Ansprüche 1 bis 6, dadurch gekennzeichnet, daß man in Gegenwart von Stickstoff oder Argon arbeitet.

8. Verfahren nach mindestens einem der Ansprüche 1 bis 7, dadurch gekennzeichnet, daß man bei Atmosphärendruck, Unter- oder Überdruck arbeitet.

## Claims

1. A process for the preparation of 2,4-dichloro-5-fluorobenzonitrile, which comprises reacting 5-bromo-2,4-dichlorofluorobenzene with about 10 to about 300 mol % of copper(I) cyanide in the presence of about 10 to about 2,000 mol % of a polar aprotic solvent, with the exception of N-methylpyrrolidone, at temperatures from about 100 to about 250°C to give 2,4-dichloro-5-fluorobenzonitrile.

2. The process as claimed in claim 1, wherein the reaction with copper(I) cyanide is carried out at temperatures from about 130 to about 190°C.

3. The process as claimed in at least one of claims 1 and 2, wherein the reaction with copper(I) cyanide is carried out in the presence of tetramethylene sulfone, dimethyl sulfoxide, tetramethylene sulfoxide, dimethylacetamide or dimethylformamide as polar aprotic solvent.

4. The process as claimed in at least one of claims 1 to 3, wherein about 50 to about 110 mol % of copper(I) cyanide are used for the reaction.

5. The process as claimed in at least one of claims 1 to 4, wherein the reaction with copper(I) cyanide takes place in the presence of about 50 to about 200 mol % of a polar aprotic solvent.

6. The process as claimed in at least one of claims 1 to 5, wherein the process is carried out in the presence of a protective gas.

7. The process as claimed in at least one of claims 1 to 6, wherein the process is carried out in the presence of nitrogen or argon.

8. The process as claimed in at least one of claims 1 to 7, wherein the process is carried out at atmospheric pressure, reduced pressure or overpressure.

## Revendications

1. Procédé de préparation du 2,4-dichloro-5-fluorobenzonitrile, caractérisé en ce qu'on fait réagir le 5-bromo-2,4-dichlorofluorobenzène avec de environ 10 à environ 300 % en moles de cyanure de cuivre(I) en présence de environ 10 à environ 2000 % en möles d'un solvant aprotique polaire, à l'exception de la N-méthylpyrrolidone, à des températures comprises entre environ 100 et environ 250°C pour donner le 2,4-dichloro-5-fluorobenzonitrile.

2. Procédé selon la revendication 1, caractérisé en ce qu'on réalise la réaction avec du cyanure de cuivre(I) à des températures comprises entre environ 130 et environ 190 °C.

3. Procédé selon au moins l'une des revendications 1 et 2, caractérisé en ce qu'on réalise la réaction avec du cyanure de cuivre(I) en présence de tétraméthylènesulfone, de diméthylsulfoxyde, de tétraméthylènesulfoxyde, de diméthylacétamide ou de diméthylformamide en tant que solvant aprotique polaire.

4. Procédé selon au moins l'une des revendications 1 à 3, caractérisé en ce qu'on met en réaction de environ 50 à environ 110 % en moles de cyanure de cuivre(I).

5. Procédé selon au moins l'une des revendications 1 à 4, caractérisé en ce qu'on réalise la réaction avec du cyanure de cuivre(I) en présence de environ 50 à environ 200 % en moles d'un solvant aprotique polaire.

6. Procédé selon au moins l'une des revendications 1 à 5, caractérisé en ce qu'on travaille en présence d'un gaz protecteur.

7. Procédé selon au moins l'une des revendications 1 à 6, caractérisé en ce qu'on travaille en présence d'azote ou d'argon.

8. Procédé selon au moins l'une des revendications 1 à 7, caractérisé en ce qu'on travaille sous pression atmosphérique, en surpression ou en dépression.
